# EUROPEAN PATENT APPLICATION

(11) **EP 1 466 635 A1**
(43) Date of publication of application: **13.10.2004**
(21) Application number: 03425221.3
(22) Date of filing: 08.04.2003
(51) Int. Cl.: A61M 1/10

(54) **Ventricular assist device and accessory therefor**

(71) Applicant: Ministero dell'Istruzione, dell'Universita e della Ricerca, 00144 Roma (IT)
(72) Inventor: Arabia, Maurizio, 00137 Roma (IT); Ercolani, Mauro, 00046 Grottaferrata (Roma) (IT); Fierli, Manlio, 00050 Pontegaleria (Roma) (IT); Minoletti, Francesco, c/o Tecnobiomedica S.p.A., 00040 Pomezia (Roma) (IT); Rinaldi, Stefano, 43100 Parma (IT); Zagara, Maurizio, 00128 Roma (IT)
(74) Representative: Bosotti, Luciano

(57) **Abstract**

The device (1) comprises a bag (3) associated to which are actuator means (5, 6) which can be selectively activated so as to enable expansion of the bag (3), with consequent filling of the bag itself by a mass of blood which flows towards the bag (3), and to produce contraction of the bag (3) with consequent expulsion from the bag itself of the mass of blood that has flowed into the bag. Associated to the bag (3) is a containment casing (2) to which a duct (7) is connected for enabling inflow and outflow of aeriform with respect to the casing (2) as a result of the movement of expansion and contraction of the bag (3). A pressure limiter (14) is provided for determining selectively and maintaining in a precise way the value of pressure of said aeriform which determines outflow - and possibly inflow - of the aeriform with respect to the aforesaid casing.

## Description

The present invention relates in general to ventricular-assist devices (VADs).

In particular, the present invention relates to an improvement of the device described in the document EP-A-1066840.

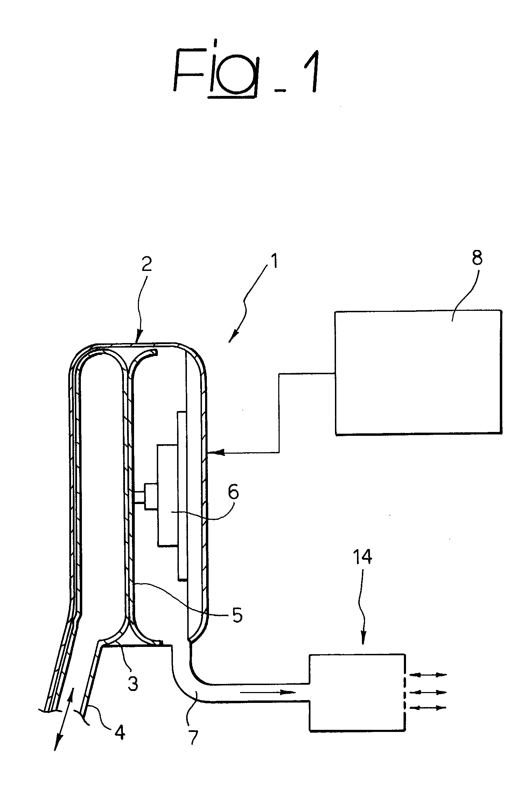

The above device corresponds substantially to the diagram illustrated in Figure 1, where the reference number 1 designates a pumping device built, for example, according to the criteria illustrated not only in the document EP-A-1066840 already mentioned previously, but also in EP-A-0728488 and EP-A-0728489.

The device 1 basically comprises a shell or casing 2 (made of biocompatible material, such as, for example, titanium) designed to be implanted in a thoracic or abdominal position. Inside the casing 2 is a deformable bag 3, made, for example, of polyurethane or of a silicone elastomer, provided with respective ducts, designated, as a whole, by 4, for inflow/outflow of the blood.

As may be inferred from the description of the European patent applications mentioned previously (to which reference may be made for an illustration of the corresponding details of implementation), there are usually present two ducts 4 designed to function, respectively, as inflow duct for inflow of the blood into the bag 3 and as outflow duct for outflow of the blood from the bag 3.

In the typical condition of implantation of the device 1, the ducts 4 are connected - by means of respective cannulae - one to an opening for drawing of the blood, said opening being made (usually in apical position) in the left ventricle of the carrier, and the other to the aorta of the carrier himself.

The ducts have associated thereto respective valves (consisting usually of cardiac valves of the prosthetic type, for example, of the type with oscillating-disk open/close element), designed to bestow upon the ducts 4 the necessary characteristics of unidirectionality as regards the flow of the blood from the left ventricle towards the bag 3 and from the bag 3 towards the aorta.

The above connection enables the bag 3 to take over the function of the left ventricle of the carrier in the process of pumping the blood towards the aorta, hence towards the circulatory system.

For achieving the above purpose, the device 1 comprises an actuator designed for enabling the bag 3, alternately:
- to expand so as to receive the blood from the left ventricle of the carrier, which in this way manages to eject the blood arriving from the cardiovascular circulation with a very small effort; and
- to contract so as to expel the blood that has collected inside the bag 3 in the direction of the aorta, thus taking over the function of the ventricle of the carrier in the process of pumping the blood towards the vascular system.

In the example illustrated, the reciprocating movement of expansion and contraction of the bag 3 is controlled by means of an actuator of an electromechanical type basically comprising a cup or plate 5, which is able to compress (in practice to squeeze) selectively the bag 3 against an opposite wall of the casing 2, and an electric motor 6 able to act on the plate 5 by means of a screw. The motor 6 is usually controlled by an electronic control device 8.

The reference number 7 designates a further duct (normally coming under a percutaneous line, when the device 1 is implanted in the body of the patient), the function of which is to enable inflow and outflow of air towards and from the internal space of the casing 2 of the device with respect to a space outside the body of the patient. The aforesaid inflow/outflow of aeriform is clearly induced by the variations of volume to which the bag 3 is subject as a result of the reciprocating movement of expansion/contraction controlled by the actuator 5, 6.

One of the solutions described in EP-A-1066840 envisages associating to the duct 7 a control device 14 the function of which is basically that of:
- enabling, in a substantially non-impeded way, inflow of the air from the outside towards the inside of the casing 2 during the stage of contraction of the bag 3; and
- countering, by means of a constriction, outflow of air from the inside of the casing 2 towards the outside.

Another embodiment illustrated in EP-A-1066840 envisages configuration of the device 14 in such a way as to associate to the bag 3 of the ventricular-assist device a further deformable bag, which is able to be set in communication with the aforesaid duct and the elastance of which - controllable, for example, by means of a spring - determines the resistance opposed by the bag of the ventricular-assist device to filling with blood.

By intervening on the device 14, the person who assists the carrier of the device 1 in the stage of recovery of the ventricular functionality may thus adopt a strategy of "weaning" (which, in effect, may be likened to a true rehabilitation to use of the cardiac muscle) by gradually increasing the effort required for the natural ventricle to drive the blood so that it flows into the bag 3.

Added to the above are the following main possibilities:
- maintaining in any case synchronization of the operation of the device 1 with the movement of expansion/contraction of the heart of the carrier; and
- intervening on the device 14 for reducing the resistance opposed by the bag 3 to filling by the ventricle of the carrier whenever it is found that the level set is too high for the degree of recovery of the ventricular functionality achieved at the moment.

The ventricular-assist device described in the document cited above is usually designed to be used for a limited period (for example, 3-4 months) so as to take over temporarily the function of the left ventricle which in the mean time is able to recover, also as a result of appropriate therapies, a level of functionality sufficient for enabling the patient to return, after removal of the VAD, to substantially normal living conditions. The solution described in the document EP-A-1066840 is designed to be employed to particular advantage in the so-called "weaning" stage, i.e., the stage in which, after the functionality of the ventricle has been taken over totally by the ventricular-assist device, in the recovery stage the ventricle is gradually brought back to carrying out its function, the aim being to arrive at removal of the ventricular-assist device when the natural ventricle has recovered a satisfactory level of functionality.

The solutions described in EP-A-1066840 enable modification and increase of the effort required for the natural ventricle to cause the blood to flow into the bag 3 of the ventricular-assist device but not to adjust it in a precise way, maintaining it constant as the heart rate and the cardiac flow vary.

In the case of the solution described in EP-A-1066840 and based upon use of a device 14 containing constrictions, the pressure upstream of said constriction, and hence the pressure against which the natural ventricle has to pump the blood, varies according to the speed of air inflow, and consequently varies as the cardiac output and the heart rate vary.

Likewise, in the variant where the device is based upon the use of a reservoir that is closed and compressed with a spring, the pressure varies as the volume of said reservoir varies and hence, once again, as the cardiac output varies.

Studies show that, for the purpose of optimizing management of the stage of weaning of the patient from the VAD, by means of a rehabilitation to use of the heart muscle based upon a progressive and controlled increase in the effort required for the natural ventricle to cause the blood to flow into the bag 3, it would be useful for the said effort not to be simply adjustable but also stable and independent of the physiological variations in the rhythm and the blood flow of the natural heart.

The purpose of the present invention is to provide a solution which will enable implementation of the above improvement.

According to present invention, the above purpose is achieved thanks to a device presenting the characteristics referred to specifically in the ensuing claims. The invention also relates to a corresponding accessory.

The invention will now be described, purely by way of non-limiting example, with reference to the annexed drawings, in which:
- Figure 1, which regards the general structure of a ventricular-assist device, and - as such - represents both the known art and the solution according to the invention, has already been described previously; and
- Figures 2 to 5 illustrate in greater detail the characteristics of a preferred embodiment of the solution according to the invention.

The solution according to the invention will now be described with reference mainly to the characteristics of the device, which is designated in the figures by the reference number 14.

It will be appreciated, in fact, that the solution according to the invention is suited for being implemented in ventricular-assist systems of various kinds, hence in a way largely independent of the nature and criteria of operation of the actuator elements used, as well as of the specific geometrical, mechanical and constructional characteristics of the bag.

Basically, the solution according to the invention envisages the use of a pressure limiter instead of the constriction set in the line of outflow of the aeriform from the casing of the ventricular-assist device as described in EP-A-1066840.

In the currently preferred embodiment of the invention, the device for adjustment of the effort that the natural ventricle must exert for filling the deformable bag of the VAD is hence, broadly speaking, made up of:
- a device which limits the pressure inside the rigid casing of the VAD keeping it lower than or equal to a maximum adjustable and/or variable value, by controlling the outflow of aeriform from the rigid casing of the VAD; and
- a valve which enables, in a non-impeded way, inflow of the air from the outside towards the inside of the casing during the stage of contraction of the bag.

Using, in a combined way, the above two devices, which are in themselves known to the art and may be made in various ways (pressure limiters and one-way valve), for controlling the flow of air in the duct 7, it is possible to regulate, in a way independent of the heart rate and of the cardiac output, the pressure necessary for filling the bag and hence the effort to which the natural ventricle is subjected. This enables, as anticipated, handling of the stage of weaning from the VAD in an optimal and reproducible way.

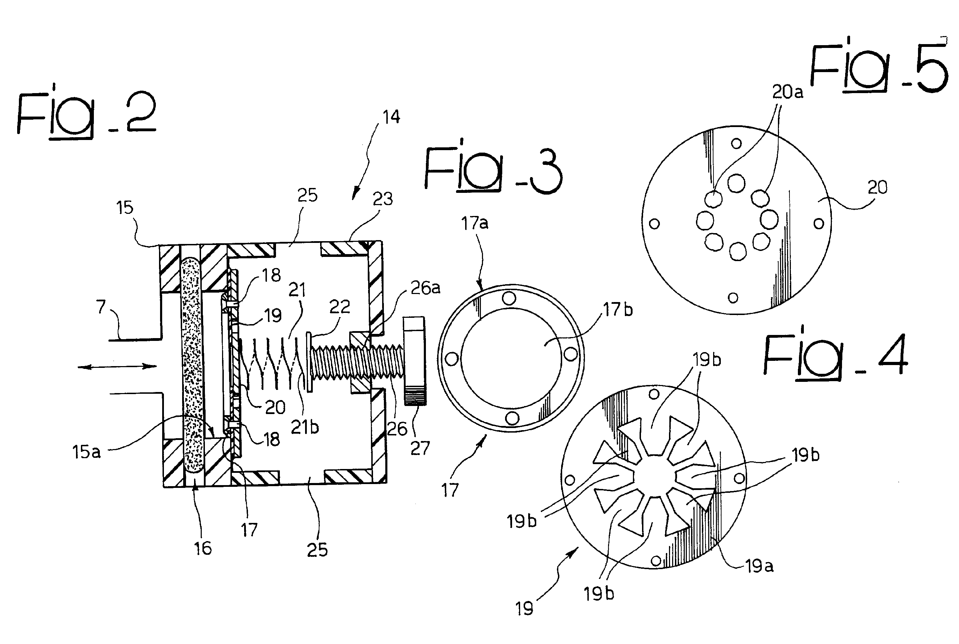

Figure 2 illustrates the characteristics of the currently preferred embodiment of the present invention.

In Figure 2, the device 14 is configured in the form of an accessory comprising a sort of plug designed to be associated to a termination 15 of the duct 7 (see Figure 1) located in a position outside the carrier's body.

Usually, the termination 15 is provided with a filter 16 (of a known type), designed for obtaining an action of purification of the air in order to prevent entry of dust or, possibly, bacteria in the direction of the casing 2.

The reference number 17 designates an annular ring nut which projects into the axial orifice 15a of the termination 15 in a position corresponding to the distal end of said orifice. The distal end is clearly understood as the end opposite to the duct 7.

The ring nut 17 forms part of an open/close element which comprises, in the example of implementation illustrated, a sandwich structure constituted by three elements connected to one another by means of fixing elements such as screws, pins or rivets 18.

The three elements of the aforesaid open/close element or assembly are the following:
- the ring nut 17, which - as may be seen more clearly in Figure 3 -, in addition to a profile or contour 17a designed to co-operate in a relationship of containment and a guide with the wall of the orifice 15a, has a central orifice 17b of a "large" size, i.e., of a size such as to enable a substantially non-impeded flow of air through the ring nut 17;
- a membrane 19 made of flexible material, such as for example silicone rubber, which - as may be seen more clearly in Figure 4 - has an external annular portion 19a, from which there extends radially, towards the centre of the membrane 19, a plurality of appendages 19b having a peninsular shape; and
- a flange 20, which - as may be seen more clearly in the view of Figure 5 - is provided with a plurality of holes 20a set in positions such that, when the assembly formed by the ring nut 17, the membrane 19 and the perforated flange 20 is assembled by means of the rivets 18, the holes 20a are in positions corresponding to the appendages 19b.

The fact that the membrane 19 is set on the side of the flange 20 facing the duct 7 means that the inflow of air from the outside towards the duct 7 may be obtained through the openings 20a not engaged by the appendages 19b which divaricate with respect to said openings under the thrust of the air which flows with a very low pressure drop.

Instead, the outflow of air in the opposite direction (i.e., from the duct 7 towards the outside) cannot be obtained through the openings 20a: the appendages 19b are, in fact, pushed against the flange 20, in a position of occlusion of the respective openings, precisely by the air which would tend to flow out of the duct 7.

The corresponding mechanism of operation is known - also for its simplicity and reliability - from numerous applications, both of an industrial type (for example, piston or membrane pumps) and in the sector of sports equipment or equipment for amusement (for example, pumps for inflating pneumatic mattresses, small watercraft, rubber dinghies, etc.).

The outflow of air from the duct 7 towards the outside of the device may hence be obtained simply by causing the assembly made up of the ring nut 17, the membrane 19 and the flange 20 (this assembly constituting in practice, as regards the outflow of air from the duct 7, a sort of plug applied so as to close the distal end of the orifice 15a of the termination 15) to undergo complete displacement so as to disengage - even just marginally - the distal opening of the duct 15a.

The reference number 21 designates an elastic element (typically constituted by a helical spring) designed to force the assembly made up of the elements 17, 19 and 20 against the distal opening of the orifice 15a of the termination 15.

For achieving the above purpose, the spring 21 is set with a first end 21a resting, usually in a central position, against the flange 20. The spring 21 has then a second end 21b, which bears upon a plate 22 held by means of a screw 26 on a part 23 of the casing of the device 14. This part of casing defines, with respect to the termination 15 and the orifice 15a, a further chamber 24 for collecting the air which communicates with the external environment through one or more openings 25 of "large" size.

Also in this case, "large" is here understood as indicating a size such as to enable a substantially non-impeded flow of air through the opening or openings 25.

The foregoing applies both as regards the inflow of air into the chamber 24 (in view of the subsequent possible inflow of the air into the duct 7 through the openings 20a left free by the appendages 19b) and as regards the outflow towards the external environment, from the chamber 24, of the air that has flowed out of the duct 7 (i.e., of the air that has come out of the axial orifice 15a of the termination 15 in the device 14, displacing the assembly formed by the parts 17, 19 and 20 from the distal opening of the orifice 15a).

The plate 22 is mounted on the free end of the screw 26, which engages an internal thread 26a provided in the body of the part of casing 23. The head part of the screw 26 projects outside the casing 23 by means of a knob 27.

By turning the knob 27, it is thus possible to move the screw 6 axially and modify the position of the plate 22, by selectively regulating the elastic force exerted by the spring 21 against the assembly constituted by the parts 17, 19 and 20.

In this way, it is therefore possible to vary selectively the force necessary for disengaging the assembly constituted by the parts 17, 19 and 20 from its resting position against the wall of the orifice 15a. Consequently, the spring 21 determines (preferably in a selective way, since the elastic force exerted is adjustable by acting on the screw 26) the level of pressure which the aeriform inside the duct 7 must reach in order to come out, and hence the maximum pressure value inside the ventricular-assist device 1.

This moreover means that the spring 21 determines (preferably in a selective way) the resistance opposed to the movement of expansion of the bag 3 of the ventricular-assist device (see Figure 1).

Exit of the aeriform 7 from the device 14 is obtained as an effect of the displacement of the assembly constituted by the parts 17, 19 and 20 with respect to the distal opening of the axial orifice 15a of the termination 15. This movement is very contained and hence such as not to produce appreciable variations in the length of the spring 21 and hence in the elastic force which the spring 21 itself opposes to the pressure exerted by the air which comes out of the duct 7.

Exit of the air from the duct 7 is therefore obtained in conditions of substantially constant pressure. In regard to the exit of the air from the duct 7, the device 14 thus far described hence behaves basically as a pressure limiter, in a way substantially independent of the speed and of the outflow rate of the air.

The above is achieved, avoiding the phenomenon - typical of the devices for flow regulation based upon a mechanism of constriction of a flow section - according to which the resistance opposed to the flow of air by the constriction of the flow of air varies - even very markedly - as a function of the rate of outflow of the air.

The solution according to the invention - based upon the use of a pressure limiter - hence enables, once the pressure has been set by acting on the screw 26, and consequently the force that the device must oppose, through the duct 7, to the movement of dilation of the bag 3 have been determined, the said pressure and the said force to be maintained substantially constant during all the movement of expansion of the bag. This is achieved without giving rise to undesirable - and in effect uncontrolled - variations linked to the rate at which the aforesaid movement of expansion is obtained.

Of course, the device 14 described above is suited for different possible variant embodiments.

For example, instead of envisaging the possibility for selectively adjusting the elastic force exerted by the spring 21 against the assembly constituted by the parts 17, 19 and 20 by intervening on the screw 26, it is possible to conceive obtaining the device 14 in a fixed configuration, i.e., with the spring 21 mounted simply between the parts 17, 19 and 20 and the casing 23, without any possibility of regulation. In this case, it is possible to obtain the device 14 in the form of an assortment of accessories, structurally identical to one another (in practice, made with the structure illustrated in Figure 2), but with springs 21 having different values of elastic force.

In the above case, instead of adjusting, as required, the elastic force of the spring 21 by acting on the screw 26 (as in the case of the embodiment illustrated in Figure 2), the operator may choose each time the accessory of the assortment to which there corresponds the value of elastic force of the spring 21, and hence the value of the resistance to deformation of the bag 3 desired at the given moment, and thus the value of the work entrusted to the natural ventricle of the carrier.

The various accessories 14, comprised in an assortment of the type described, and characterized by different limit values of the maximum pressure acting inside the ventricular-assist device, may be made with different colours and/or shapes, so as to prove readily identifiable according to the needs of use even by not particularly expert personnel.

Should the patient evince any difficulty linked to the fact that the load set to the bag 3 is excessive for the level of recovery of the ventricular functionality so far achieved, simple removal of the device 14, configured as an accessory, enables restoring the device to the normal operating conditions corresponding to the minimum resistance of filling of the bag 3 and hence to the maximum efficiency of the ventricular assistance.

The device 14 may also be built possibly in such a way as to be mounted on the termination 15 of the duct 7 according to an arrangement exactly opposite to the one to which reference is made in Figure 2, i.e., in such a way that the device 14 operates so as not to pose an obstacle to the outflow of air from the casing 2 towards the outside environment, and instead opposing to the inflow of air from the outside environment towards the casing 2.

This movement of inflow of air from outside is induced when the bag 4 contracts under the action of the motor 6. The action of the motor is a strong action, in effect not impeded by the resistance to the flow of air through the duct 7 exerted by the accessory 14, mounted so to speak "the other way round". The net result is to obtain that, at the end of the compression stroke of the bag 3 by the plate 5, within the casing 2 there will prevail a level of pressure at least marginally lower (also in this case determined in a precise way by the value of the reaction exerted by the spring 21 on the assembly constituted by the parts 17, 19 and 20) with respect to the level of atmospheric pressure. In these conditions, when the motor 6 reverses its direction of operation, recalling the plate 5 so as to disengage the bag 3, the aforesaid level of subatmospheric pressure acts so as to favour dilation of the bag 3, and hence favour inflow of the blood from the ventricle of the carrier towards the bag 3.

In the above case, the action of selective modification of the resistance to filling exerted by the bag acts in a direction, so to speak, opposite to the one described previously. In other words, when the device 14 is mounted "the other way round", the overall effect is that of increasing the level of assistance to the ventricular function offered by the device 1 beyond the level afforded by the normal conditions of use. An arrangement of assemblage of this kind may hence be used, for example, in the period immediately following upon implantation of the VAD when, both as a consequence of the illness which has determined the need for assisting the natural heart and, possibly, owing to the traumatic effect of the surgical operation, the contractility of the natural heart is so small that it renders filling of the bag 3 by the natural heart problematical even only as a result of the modest resistance to the flow of blood determined by the cannula which connects the natural heart to the VAD, or else in the presence of crises which might arise during weaning. At the same time, the characteristics of extreme simplicity of use described previously are maintained, thus enabling rapid and safe intervention even by personnel not specifically trained in the field.

Of course, without prejudice to the principle of the invention, the details of construction and the embodiments may vary widely with respect to what is described and illustrated herein, without thereby departing from the scope of the present invention, as this is defined in the ensuing claims.

## Claims

1. A ventricular-assist device (1) comprising a bag (3), which is selectively able to:
- expand so as to enable filling of the bag (3) with a mass of blood flowing towards the bag (3) itself; and
- contract so as to expel from the bag (3) a mass of blood that has flowed into the bag (3) itself,
there being associated to said bag (3) a containment casing (2), to which a duct (7) is connected for enabling inflow and outflow of aeriform with respect to said casing (2) as a result of the movement of expansion and contraction of said bag, the device likewise comprising an element (14) for selectively regulating the passage of aeriform through said duct (7) in at least one direction of flow,
said device being **characterized in that** said element (14) comprises a pressure limiter.

2. The device according to Claim 1, **characterized in that** said pressure limiter (14) is configured for determining the value of pressure of said aeriform able to cause passage of the aeriform itself through said duct (7) in said at least one direction of flow, so that the action of selective regulation of the passage of aeriform through said duct (7) in said at least one direction of flow is independent of the rate of said flow.

3. The device according to Claim 1 or Claim 2, **characterized in that** said element (14) comprises a valve (19b, 20a), which enables in a substantially non-impeded way the passage of aeriform through said duct (7) in a direction opposite to said at least one direction of flow.

4. The device according to any one of Claims 1 to 3, **characterized in that** said pressure limiter (14) carries associated thereto regulation means (26) for varying selectively the corresponding value of limitation.

5. The device according to any one of Claims 1 to 4, **characterized in that** said pressure limiter comprises:
- an orifice (15a) defining a duct for flow of said aeriform;
- an open/close element (17, 19, 20), being able to assume a position of occlusion of said orifice (15a) and of displacing with respect to said position of occlusion under the thrust of said aeriform, enabling passage thereof through said pressure limiter in a first direction only when the pressure of said aeriform reaches a pre-determined level; and
- a valve structure (19b, 20a), associated to said open/close element and being such as to enable selectively flow of said aeriform through said open/close element in a second direction of flow of said aeriform, opposite to said first direction of flow, when said open/close element (17, 19, 20) is in a position of occlusion of said orifice (15a).

6. The device according to Claim 5, **characterized in that** said open/close element (17, 19, 20) carries associated thereto an elastic element (21) which forces the open/close element (17, 19, 20) in a position of occlusion of said orifice (15a), the arrangement being such that the elastic force exerted by said elastic element (21) determines the value of pressure of said aeriform able to cause displacement of said open/close element (17, 19, 20) from the position of occlusion of said orifice (15a).

7. The device according to Claim 6, **characterized in that** said elastic element (21) is a helical spring.

8. The device according to Claim 4 and either Claim 6 or Claim 7, **characterized in that** said elastic element (21) carries associated thereto an adjustment member (26, 27) for varying selectively the degree of the elastic force exerted by said elastic element (21) on said open/close element (17, 19, 20).

9. The device according to Claim 8, **characterized in that** said adjustment member comprises a screw (26).

10. The device according to Claim 8 or Claim 9, **characterized in that** said adjustment member (26) carries associated thereto an actuating formation (27) accessible from the outside of the device.

11. The device according to any one of Claims 5 to 10, **characterized in that** said valve structure (19b, 20a) comprises at least one flow opening for said aeriform (20a) and one diaphragm element (19b) selectively translatable between a position of occlusion of said at least one opening (20a) and a position of disengagement with respect to said at least one opening (20a) as a result of the pressure exerted on said at least one diaphragm element (19b) by said aeriform.

12. The device according to any one of Claims 5 to 11, **characterized in that** said open/close element comprises:
- a ring nut (17) provided with at least one opening (17b) for non-impeded passage of said aeriform, said ring nut (17) being able to co-operate with the wall of said at least one orifice (15a);
- a membrane (19) comprising said at least one diaphragm element (19a); and
- a flange (20) provided with said at least one opening (20a);
said ring nut (17), said membrane (19), and said flange (20) being connected to one another according to a general sandwich arrangement.

13. The device according to any one of Claims 1 to 12, **characterized in that** said pressure limiter (14) acts so as to determine the pressure at which the outflow of said aeriform from said casing (2) through said duct is obtained.

14. An accessory which can be associated to a device according to any one of Claims 1 to 13, **characterized in that** it incorporates said pressure limiter (14).

15. The accessory according to Claim 14, **characterized in that** said accessory (14) can be associated to said duct (7) in a selectively removable way.

16. The accessory according to Claim 14 or Claim 15, **characterized in that** it is comprised in an assortment of homologous accessories (14) having values of intervention of the pressure limiter different from one another.

17. The accessory according to Claim 16, **characterized in that** the accessories of said assortment present at least one respective characteristic which differentiates them.

18. The accessory according to Claim 16, **characterized in that** the accessories of said assortment present respective different colours and/or shapes.

19. The accessory according to any one of Claims 14 to 18, **characterized in that** the accessory (14) is configured so that it can be associated to said duct (7) in a position such that said pressure limiter (14) acts so as to determine the pressure at which the inflow of said aeriform towards said casing (2) through said duct is determined.
